Europäisches Patentamt

European Patent Office

Office européen des brevets

(11) Veröffentlichungsnummer: **0 016 343**
**B1**

(12) # EUROPÄISCHE PATENTSCHRIFT

(45) Veröffentlichungstag der Patentschrift:
18.05.83

(51) Int. Cl.³: **A 61 M 5/20**

(21) Anmeldenummer: **80100810.3**

(22) Anmeldetag: **18.02.80**

(54) **Gerät für Dauerinfusionen.**

(30) Priorität: **22.02.79 DE 2906830**

(43) Veröffentlichungstag der Anmeldung:
**01.10.80 Patentblatt 80/20**

(45) Bekanntmachung des Hinweises auf die Patenterteilung:
**18.05.83 Patentblatt 83/20**

(84) Benannte Vertragsstaaten:
**AT BE CH DE FR GB IT LU NL SE**

(56) Entgegenhaltungen:
**DE-A-2 112 654**
**DE-A-2 529 066**
**GB-A-1 026 593**
**US-A-3 886 938**
**US-A-4 059 110**

(73) Patentinhaber: **Intermedicat GmbH, Gerliswilstrasse 45,
CH-6020 Emmenbrücke (CH)**

(72) Erfinder: **Hessberg, Sigfried, Zur Kroneneiche 14,
Melsungen (DE)**
Erfinder: **Dold, Werner, Wallfahrtsstrasse 20, Triberg
(DE)**

(74) Vertreter: **von Kreisler, Alek, Dipl.-Chem. et al,
Deichmannhaus am Hauptbahnhof, D-5000 Köln 1 (DE)**

ACTORUM AG

Gerät für Dauerinfusionen

Die Erfindung bezieht sich auf ein Gerät für Dauerinfusionen, dessen Laufwerk zum Antrieb einer Injektionsspritze einen Federmechanismus zum Vorschub des Spritzenstössels und ein mechanisches Uhrwerk zur Steuerung des Stösselvorschubes aufweist.

Zur Behandlung vieler Krankheiten ist es erforderlich, kleine Dosen hochwirksamer körpereigener chemischer Substanzen und Arzneimittel über einen längeren Zeitraum kontinuierlich in die Blutbahn eines Patienten abzugeben. Es ist bekannt, für derartige Dauerinjektionen Laufwerke zum Antrieb von Injektionsspritzen zu verwenden, die in kleinen Gehäusen untergebracht sind, die am Patienten befestigt werden können. Bekannte Laufwerke arbeiten mit einem Elektromotor mit Batterieantrieb. Über ein Getriebe wird eine Mutter auf einem Gewinde in Drehbewegung versetzt, und es wird die lineare Bewegung der Mutter auf den Kolben einer Injektionsspritze übertragen. Ein Uhrwerk fehlt bei dieser bekannten Konstruktion. Die Vorschubsteuerung ergibt sich durch die Art des Getriebes, und für verschiedene Infusionszeiten werden jeweils gesonderte Geräteausführungen benötigt. Zur Einstellung der Dosisrate ist jeweils eine besondere Anpassung der Fliessfähigkeit des zu injizierenden Mittels erforderlich. Eine gewisse Unzuverlässigkeit ergibt sich bei diesem Gerät durch die Abhängigkeit von dem Ladungszustand der Batterie. Bei unvorhergesehenem Batterieausfall wird die Injektion sofort unterbrochen. Der Anschluss eines solchen Gerätes an ein Stromnetz kann zwar diese Unsicherheit beheben, jedoch ist der Patient in diesem Falle für die Dauer der Injektion stadortgebunden. Im übrigen hat sich gezeigt, dass jede Einleitung von Elektrizität in den lebendigen Körper selbst in der Grössenordnung von Mikroampère Herzbeschwerden verursacht.

Um die mit einem elektrischen Antrieb verbundenen Nachteile zu vermeiden, sind mechanische Laufwerke zum Antrieb von Injektionsspritzen für Dauerinfusionen entwickelt worden, bei denen zum Vorschub des Spritzenstössels ein Federmechanismus dient und der Vorschub von einem mechanischen Uhrwerk gesteuert wird (DE-A-2 112 654 und US-A-3 886 938).

Das Gerät nach der erstgenannten Druckschrift ist mit einem Betätigungsmechanismus ausgestattet, der ein Uhrwerk umfasst, welches einen Schieber längs einer Spindel antreibt, wobei der Schieber mit einer gezahnten unteren Kante mit einem Getrieberad des Uhrwerkes in Eingriff stehen kann und mit einem Stift versehen ist, der mit dem Spritzenstössel zusammengreift und ihn verschiebt.

Zum Einlegen der gefüllten Spritze und zum Aufziehen des Uhrwerkes werden der Schieber und das Getrieberad des Uhrwerkes ausser Eingriff gebracht, so dass der Schieber frei verschoben werden kann. Sodann wird das Uhrwerk mit dem Schieber gekoppelt. Danach drückt das Uhrwerk den Kolben langsam in die Spritze ein, wodurch das Medikament aus der Spritze gedrückt wird. In diesem Falle wird auch der Spritzenstössel von der Triebfeder des Uhrwerkes angetrieben, so dass nur ein einziger Kraftspeicher und damit auch nur einziger Kraftfluss vorhanden ist. Wenn Schieber und Getrieberad des Uhrwerkes sich ausser Eingriff befinden, lastet – da die Unruhe mechanisch gestoppt wird – die volle Kraft des Federhauses auf dem Uhrwerk und kann dieses beschädigen. Ausserdem wirken sich Änderungen der Last, d.h. der durch die Viskosität des zu injizierenden Mittels und den Kanalquerschnitt von Leitungen und Kanülen sowie innere Reibkräfte der Spritze bedingten Kräfte zur Betätigung der Spritze, voll als Variation der für das Uhrwerk verbleibenden Antriebskraft aus. Dies hat zur Folge, dass bei schwergängigen Spritzen und/oder hohem Flüssigkeitsgegendruck (hohe Viskosität, enge Leitung und Kanüle) die Kraft zu null werden kann, die das Uhrwerk und den Spritzenstössel antreiben soll; das Gerät bleibt stehen und erfüllt seinen medizinischen Zweck nicht. Ist die Spritze leichtgängig, so verbleibt für das Uhrwerk so viel Antriebskraft, dass der Anker bzw. die Unruhe durch Prellschwingungen stark gefährdet sind. Das kann zur plötzlichen Zerstörung führen, setzt aber zumindest die Lebensdauer des Gerätes beträchtlich herab, wie die Praxis gezeigt hat. Ferner wird die Genauigkeit der Zeitsteuerung beeinträchtigt.

Das andere mechanisch arbeitende Gerät für Dauerinfusionen (US-A-3 886 938) weist demgegenüber zwar zwei Kraftspeicher in Form von zwei Schraubenfedern einerseits und einer Hauptfeder anderseits auf, jedoch sind die Kraftflüsse der beiden Kraftspeicher nicht getrennt. Die Federkraft wird über eine in dem Gerätegehäuse verschiebbar angeordnete Basis und eine Zahnstange auf den Spritzenstössel übertragen, damit er sich in Vorschubrichtung bewegt. Dabei soll das mit der Hauptfeder ausgestattete Uhrwerk über ein Ritzel die Vorschubgeschwindigkeit der Zahnstange steuern. Diese beiden Federmittel stehen über das Ritzel in dauernder kraftschlüssiger Verbindung, so dass eine dauernde gegenseitige Beeinflussung stattfindet. Dies wirkt sich so aus, dass eines der beiden Federmittel überflüssig ist, weil beide nur einen einzigen gemeinsamen Kraftfluss erzeugen. Die dauernde Verbindung der beiden Federmittel hat eine Rückwirkung von den beiden Schraubenfedern auf den Uhrwerkmechanismus zur Folge, weshalb die Hauptfeder auch die Zahnstange und die Schraubenfedern auch das Uhrwerk antreiben und ohne weiteres durch ein einziges Federmittel ersetzt werden können. Trotz des Vorhandenseins von zwei Federmitteln (Kraftspeichern) reduziert sich somit dieses Gerät auf den Stand des Gerätes nach DE-A-2 112 654, dessen einer einziger Kraftfluss bewirkt, dass die inneren Reibkräfte der jeweiligen Injektionsspritze die Steuerung des Stösselvorschubes durch das Uhrwerk verfälschen, so dass ein gleichmässiger Stösselvorschub nicht erreicht wird.

Der Erfindung liegt die Aufgabe zugrunde, ein Gerät für Dauerinfusionen nach US-A-3 886 938 so zu verbessern, dass der Spritzenstössel unabhängig von den Reibkräften in der Spritze zeitgesteuert jeweils absolut gleichmässig vorgeschoben wird, so dass bei jeder Art der Spritze, des Injektionsmittels und der Kanüle bzw. der Leitung ein präzise gesteuerter Stösselvorschub erreicht wird.

Diese Aufgabe wird dadurch gelöst, dass das Uhrwerk den Federmechanismus über Schrittschaltmittel steuert, die die Kraftflüsse des Federmechanismus und des Uhrwerkes voneinander trennen.

Diese beiden Kraftflüsse erzeugen voneinander getrennte Bewegungsabläufe, durch die sowohl ein gleichmässiger Lauf des Uhrwerkes als auch ein gleichmässiger Antrieb des Stössels der Injektionsspritze gewährleistet sind. Ein ungleichmässiger Spritzendruck spielt keine Rolle mehr. Jede beliebige Spritze wird mit der gleichen Präzision über einen gewünschten Zeitraum vollständig entleert. Die auf den Stössel der Injektionsspritze übertragene Federkraft bleibt konstant. Das Uhrwerk übernimmt keine Antriebsfunktionen für den Stössel, sondern hat lediglich die Aufgabe, den Stösselvorschub über die Schrittschaltmittel in bestimmten Abständen festzuhalten und freizugeben, wobei keine Rückwirkung von dem Federmechanismus zum Stösselvorschub auf das Uhrwerk auftritt. Daher ergibt sich eine sehr präzise Uhrwerksteuerung für einen gleichmässigen Stösselvorschub.

Vorteilhafterweise ist vorgesehen, dass der Federmechanismus und das Uhrwerk mit je einer Spiralzugfeder und Räderwerkübersetzung ausgestattet sind und dass die beiden Spiralzugfedern koaxial auf einer Federwelle montiert und mit einer gemeinsamen Aufzugswelle verbunden sind. Es werden stets beide Spiralzugfedern gleichzeitig aufgezogen und als Aufzugsmittel dient eine an einem eine Auflage für die Injektionsspritze aufweisenden Gehäuse schwenkbare Abdeckung für die Spritzenauflage. Die schwenkbare Abdeckung ist mit der Aufzugswelle drehfest verbunden. Da die Abdeckung die Spritzenauflage in dem Gehäuse abdeckt, ist es für den Zugang zu der Spritzenauflage erforderlich, die Abdeckung zu verschwenken und hierbei werden die beiden Spiralzugfedern aufgezogen. Damit der Benutzer die beiden Spiralzugfedern vor Inbetriebnahme des Gerätes jeweils vollständig aufzieht, ist eine Sicherung gegen vorzeitiges Schliessen der Abdeckung vorgesehen. Diese besteht aus einer mit der Abdeckung fest verbundenen, schrägverzahnten Scheibe, die mit einer Federklinke derart zusammenwirkt, dass die Abdeckung erst nach ihrer Schwenkung um 180° schliessbar ist. So kann die Abdeckung nur geschlossen werden, wenn der Vortriebsmechanismus und das Uhrwerk vollständig aufgezogen sind, also die Spiralzugfedern voll auf 180° gespannt wurden. Zur Ausrastung der Federklinke in der End-Schwenkstellung der Abdeckung dient eine von einem Mitnehmerzapfen an der schrägverzahnten Scheibe betätigte Nockenfläche. Ein Öffnen der Abdeckung zum Herausnehmen der Spritze ist vor Ablauf der Gesamtinfusionszeit jederzeit möglich. Die Spritze kann beliebig aus der Spritzenauflage des Gehäuses herausgenommen werden.

In vorteilhafter Ausgestaltung der Erfindung ist vorgesehen, dass der Federmechanismus über eine Übersetzung ein Ritzel antreibt, das eine Zahnstange vorschiebt, die einen an den Stössel der Spritze angreifenden Arm aufweist und dass auf der Welle des Sekundenrades des Uhrwerks ein Exzenter angeordnet ist, der einen auf der Ankerwelle sitzenden Hemmanker betreibt, welcher auf ein von dem Federmechanismus angetriebenes Schaltrad wirkt, das über die Übersetzung das Ritzel steuert. Der Hemmanker sperrt jeweils die letzte Getriebestufe des Vorschubwerkes und das Schaltrad steuert durch Übertragung von Halteimpulsen auf den Federmechanismus den allein von diesem hervorgerufenen Vorschub des Stössels der Injektionsspritze. Bedingt durch die Umdrehung des Sekundenrades wird die Getriebestufe, auf der das Schaltrad sitzt, pro Zeiteinheit beliebig oft freigegeben und jedesmal wird von dem Federmechanismus ein Vorschubstoss auf die Zahnstange ausgeübt. Es ergibt sich ein allmählicher gleichmässiger Ausstoss des Spritzeninhaltes unabhängig von der Beschaffenheit des zu injizierenden Präparates und der Schwergängigkeit der Spritze. Der einfache Aufbau der Anordnung zur Übertragung der Zeitsteuerung von dem Uhrwerk auf den Stösselvorschub garantiert eine zuverlässige Funktion der Steuerung. Durch bestimmte Auslegung des Hemmankers und des Schaltrades ist es möglich, verschiedene Bewegungsabläufe zu steuern.

Vorteilhafterweise ist das Schaltrad als Flügelrad mit symmetrisch angeordneten Flügeln ausgebildet. Es ist zweckmässig, dass das Schaltrad vier Flügel mit 90° Abstand aufweist, und dass der Hemmanker mit zwei hintereinanderliegenden Anschlagnasen für die Flügel des Schaltrades versehen ist. Zur Einstellung verschiedener Vorschubgeschwindigkeiten des Spritzenstössels ist vorgesehen, dass die Breite der Flügel zueinander unterschiedlich ist und dass der Hemmanker zur Erfassung von Flügeln unterschiedlicher Breite auf der Ankerwelle axial verstellbar angeordnet ist. Das Uhrwerk bleibt von dieser Steuerung unbeeinflusst und läuft immer mit der gleichen Laufzeit ab. Zur Erzielung langer Infusionszeiten wird der Hemmanker relativ zu dem Schaltrad so eingestellt, dass er pro Zeiteinheit möglichst viele Flügel des Schaltrades hemmt, so dass die Vorschubbewegung des Spritzenstössels in kurze Schritte aufgeteilt ist, wodurch sich die Gesamtvorschubzeit verlängert. Entsprechend wird zur Verkürzung der Gesamtvorschubzeit die Schrittlänge vergrössert, indem der Hemmanker pro Zeiteinheit mit einer geringeren Anzahl von Flügeln des Schaltrades zusammengreift, um das Schaltrad pro Zeiteinheit seltener anzuhalten.

Zur axialen Verstellung des Hemmankers dient vorteilhaft eine gewellte Kurvenscheibe, die in einen Querschlitz an der Ankerwelle eingreift. Die Stellung der Kurvenscheibe kann mittels eines Betätigungsteiles auf der Aussenfläche des Gehäuses einstellbar und durch eine Rastvorrichtung gesichert sein. Der Benutzer kann selbst ohne Schwierigkeiten die Infusionszeit wählen, wobei eine Umschaltung während des Ablaufes möglich ist.

Eine weitere vorteilhafte Ausgestaltung der Erfindung besteht darin, dass die Zahnstange zwischen einem Stangenpaar geführt ist, das an einem Kipphebel befestigt ist, dessen eines Ende schwenkbar mit dem Gehäuse verbunden ist und dessen anderes Ende unter der Wirkung einer Zugfeder und einer mit der Aufzugswelle drehfest verbundenen Nockenscheibe steht. Hierdurch wird erreicht, dass bei aufgeklappter Abdeckung die als Vorschubeinheit für den Spritzenstössel wirksame Zahnstange automatisch ausser Eingriff mit dem Vorschubwerk gebracht wird. Die Zahnstange ist frei verschiebbar und der Antrieb unwirksam, so dass die Spritze aus dem Gehäuse herausgenommen und wieder voll aufgezogen beziehungsweise entlüftet oder zur Erreichung einer anderen Stellung bei geöffnetem Gehäuse von Hand betätigt werden kann. Bei in das Gerät eingesetzter Spritze und geöffneter Abdeckung kann das Uhrwerk ablaufen, ohne dass die Spritze betätigt wird, da der Vorschub ausgekuppelt ist.

Mit der Bewegung der Gehäuseabdeckung sind gemäss der Erfindung insgesamt drei Funktionen gekoppelt, und zwar der Aufzug der beiden Spiralzugfedern, die Sicherung gegen ein Schliessen der Abdeckung vor vollständigem Aufzug der beiden Gehwerke und die Auskupplung des Vorschubwerkes bei geöffneter Abdeckung.

In der Zeichnung ist ein Ausführungsbeispiel der Erfindung schematisch dargestellt. Es zeigt:

Fig. 1 eine schaubildliche Ansicht des Gerätes mit eingesetzter Injektionsspritze in geöffnetem Zustand,

Fig. 2 eine Draufsicht des Vorschubwerkes bei abgenommenem Gehäusedeckel,

Fig. 3 eine Ansicht des Vorschubwerkes und des Uhrwerkes, gesehen in Richtung des Pfeiles A in Fig. 2,

Fig. 3a die Einrichtung zum Aus- bzw. Einkuppeln des Vorschubwerkes mit der Zahnstange in vergrössertem Massstab,

Fig. 4 das Schaltrad mit einem Teil des Hemmankers in vergrössertem Massstab,

Fig. 5 einen Einblick in das Gehäuse, gesehen in Richtung des Pfeiles B in Fig. 3,

Fig. 6 einen Schnitt längs der Linie VI–VI in Fig. 5,

Fig. 7 die Scheibenanordnung nach Fig. 6 in auseinandergezogenem Zustand,

Fig. 8 die mittlere Scheibe der Scheibenanordnung nach Fig. 7 in anderer Stellung.

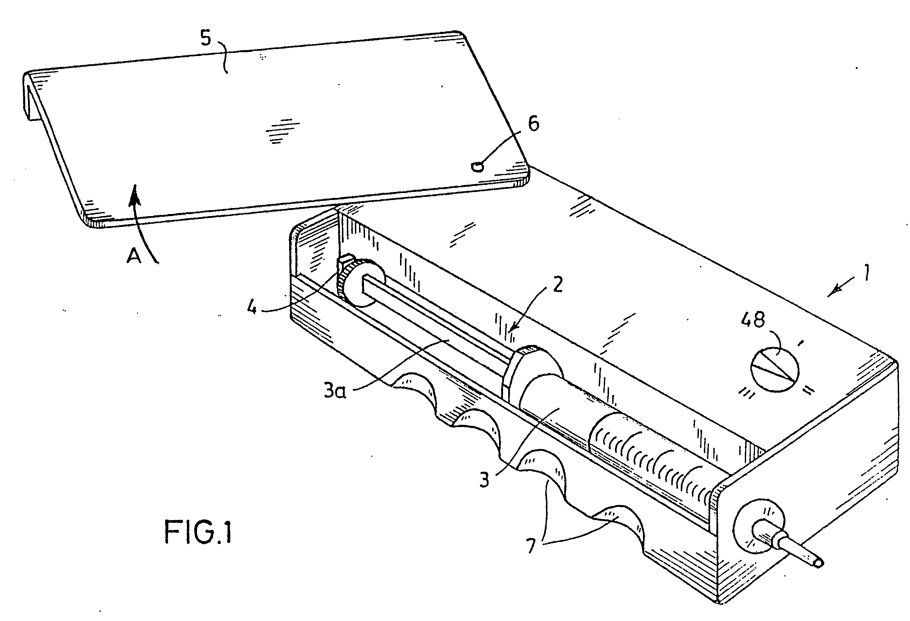

Das Gerät für Dauerinfusionen besteht gemäss Fig. 1 aus einem Gehäuse 1, das eine zur Seite hin offene Auflage 2 für eine Injektionsspritze 3, z.B. eine Einmal-Spritze aufweist. In die Spritzenauflage 2 ragt ein Arm 4 einer innerhalb des Gehäuses mit einem Vorschubantrieb koppelbaren Zahnstange. Der Arm 4 drückt gegen einen Stössel 3A der Injektionsspritze 3. Zum Verschluss der Spritzenauflage 2 dient eine Abdeckung 5. Diese ist als Winkelplatte gestaltet und bei 6 schwenkbar an dem Gehäuse gelagert. Der Drehpunkt der Abdeckung 5 ist so an dem Gehäuse angeordnet, dass immer das vordere Ende der Injektionsspritze 3 zuerst freigegeben wird, wenn die Abdeckung 5 zur Öffnung in Richtung des Pfeiles A verschwenkt wird. Fingerausnehmungen 7 erleichtern die Handhabung des Gerätes.

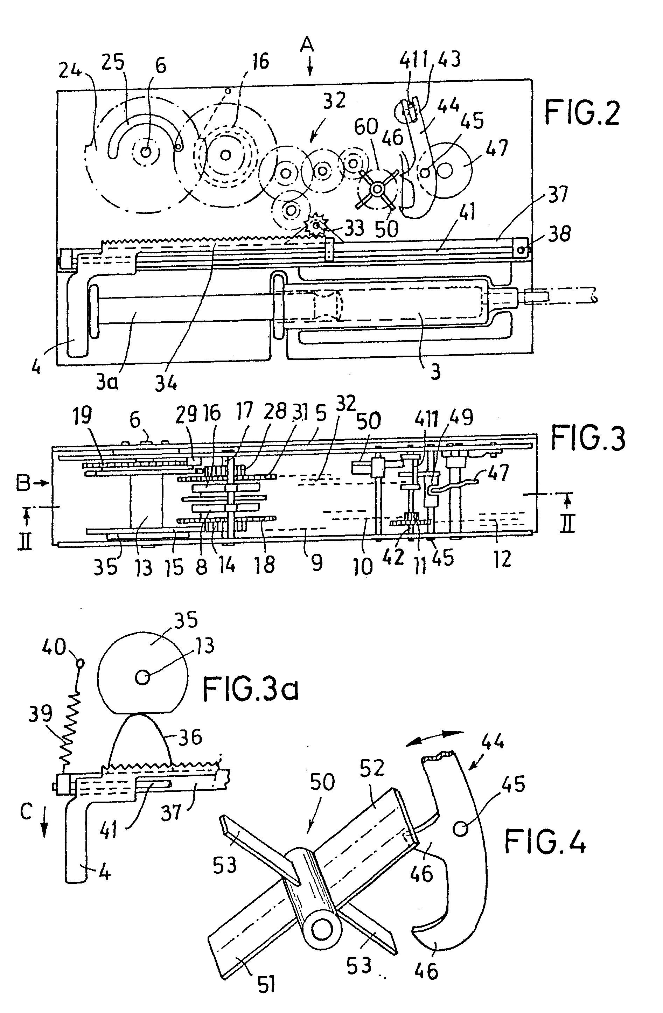

Gemäss der Erfindung zeichnet sich das Gerät dadurch aus, dass für das Uhrwerk und für das Vorschubwerk jeweils ein getrennter Kraftfluss vorgesehen ist. Zu diesem Zweck ist der Mechanismus des Uhrwerkes auf einer Seite der Längsmittelebene des Gehäuses angeordnet, während sich der Mechanismus des Vorschubwerkes auf der anderen Seite der Längsmittelebene des Gehäuses befindet (Fig. 3).

Das Uhrwerk befindet sich bei der Darstellung nach Fig. 3 auf der Unterseite des Gehäuses. Es besteht in üblicher Weise aus einer Spiralzugfeder 8, deren Kraft über ein Zahnrad 18, ein Minutenrad 9, ein Kleinbodenrad 10 und ein Sekundenrad 11 auf einen Gang 12 übertragen wird. Die Spiralzugfeder 8 steht über ein Zahnrad 14 und eine Teilzahnscheibe 15 mit einer Aufzugswelle 13 in Verbindung. Die Aufzugswelle 13 ist bei 6 drehfest mit der Abdeckung 5 des Gehäuses verbunden, so dass bei Verschwenkung der Abdeckung 5 die Spiralzugfeder 8 aufgezogen wird.

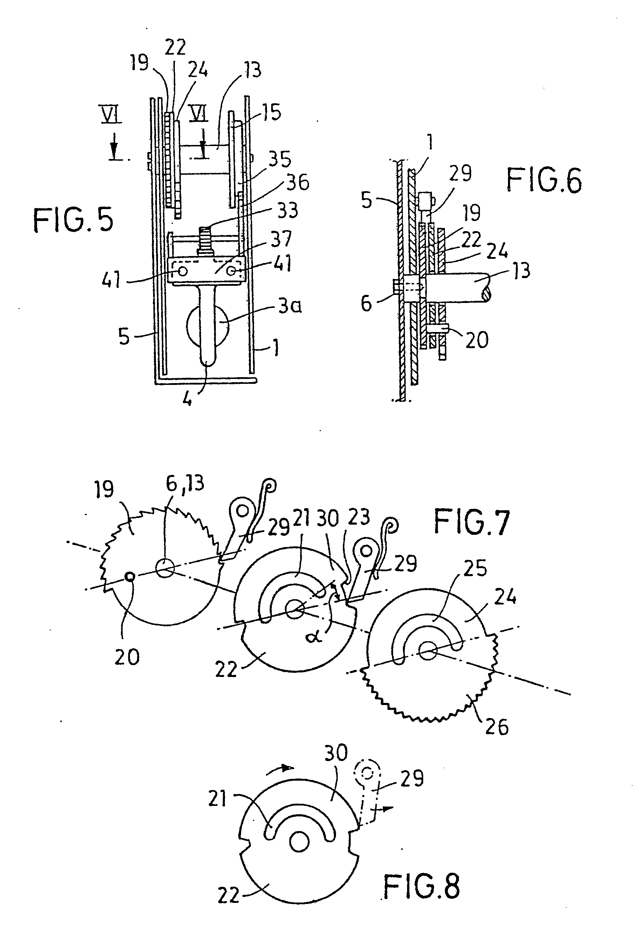

Die Aufzugswelle 13 wirkt auch auf eine dem Vorschubwerk zugeordnete Spiralzugfeder 16, die koaxial zu der Spiralzugfeder 8 auf einer Welle 17 angeordnet ist. Beide Spiralzugfedern 8 und 16 werden auf diese Weise bei Verschwenken der Abdeckung 5 gleichmässig aufgezogen. Um sicherzustellen, dass der Deckel immer um 180° verschwenkt wird, damit sowohl das Uhrwerk als auch das Vorschubwerk stets voll gespannt ist, ist die in den Fig. 6–8 veranschaulichte Scheibenanordnung vorgesehen.

Eine teilweise schrägverzahnte Scheibe 19 ist fest mit der Abdeckung 5 verbunden, so dass sie sich bei Verschwenkung der Abdeckung 5 mitbewegt. Diese schrägverzahnte Scheibe 19 trägt einen quergerichteten Zapfen 20, der durch einen Bogenschlitz 21 in einer zweiten Scheibe 22 hindurchragt, die nur um ein kleines Winkelstück drehbar ist und eine umfangsmässige Aussparung 23 aufweist. Koaxial zu diesen beiden Scheiben 19 und 22 ist eine dritte teilverzahnte Scheibe 24 vorgesehen. In dieser ist ein Bogenschlitz 25 ausgebildet, der etwas länger als der Bogenschlitz 21 der Scheibe 22 ist und durch den ebenfalls der Zapfen 20 hindurchragt. Beim Verschwenken der Abdeckung 5 zum Aufziehen des Uhrwerkes nimmt der Zapfen 20 die Scheibe 24 mit, und ihr Zahnsegment 26 überträgt die Schwenkbewegung der Abdeckung 5 über ein Zahnrad 28 (Fig. 3) auf die Welle 17, die die beiden Spiralzugfedern 8 und 16 trägt. In jeder Phase der Öffnungsverschwenkung der Abdeckung 5 sperrt der Zusammengriff der Schrägverzahnung der Scheibe 19 und der Aussparung 23 in der Scheibe 22 mit einer federbelasteten Klinke 29 ein Zurückdrehen der Abdeckung 5 in die geschlossene Stellung. Erst bei Verschwenkung um 180° und dementsprechenden vollen Aufzug der beiden Spiralzugfedern wird die Klinke 29 durch die Nockenfläche 30 der Scheibe 22 ausgerastet und die Abdeckung kann frei in die Schliessstellung zurückgeschwenkt werden. Die Drehbewegung der Scheibe 22 um einen kleinen Winkel α ist dadurch möglich, dass der Bogenschlitz 25 der Scheibe 24 etwas länger ist als der Bogenschlitz 21 der Scheibe 22.

Die Kraft der Spiralzugfeder 16 des Vorschubwerkes wird über ein Zahnrad 31 und eine nur andeutungsweise gezeichnete Übersetzung 32 auf ein Ritzel 33 übertragen, das mit einer Zahnstange 34 zusammenwirkt, deren Arm 4 gegen den Stössel 3A der Injektionsspritze 3 angreift.

Falls die Spritze 3 vor Ablauf der Gesamtinfusionszeit aus irgendwelchen Gründen aus der Spritzenauflage 2 herausgenommen werden soll, ist es zweckmässig, den Vorschub der Zahnstange 34 bis zum Wiedereinsetzen der Spritze 3 zu unterbrechen. Dies geschieht, indem bei aufgeklappter Abdeckung 5 die Zahnstange 34 automatisch ausser Eingriff mit dem Ritzel 33 gebracht wird. Zu diesem Zweck dient die in Fig. 3A herausgezeichnet veranschaulichte Einrichtung. Eine Nockenscheibe 35, die mit der Abdeckung 5 drehfest verbunden ist, wirkt über einen Federbügel 36 oder dergleichen auf einen Kipphebel 37, der als Träger für die Zahnstange 34 dient. Das eine Ende des Kipphebels 37 ist bei 38 schwenkbar mit dem Gehäuse 1 verbunden (Fig. 2), während sein anderes Ende unter der Wirkung einer Schraubenfeder 39 steht, die bei 40 fest mit dem Gehäuse verbunden ist (Fig. 3A). Die Zahnstange 34 ist zwischen zwei Stangen 41, von denen nur eine in Fig. 2 veranschaulicht ist, geführt, deren Enden an dem Kipphebel 37 befestigt sind. Bei geschlossenem Deckel ergibt sich die in Fig. 3A gezeigte Stellung, in der die Abflachung der Nockenscheibe 35 über den Bügel 36 den Kipphebel 37 in einer Stellung hält, in der das Vorschubwerk mit der Zahnstange 34 gekoppelt ist. Wird die Abdeckung 5 geöffnet, so drückt die Rundung der Nockenscheibe 35 gegen den Bügel 36, und gegen die Wirkung der Schraubenfeder 39 bewegt sich der Kipphebel 37 in Richtung der Pfeiles C in Fig. 3A, wodurch das Vorschubwerk ausgekuppelt wird.

Das eingekuppelte Vorschubwerk wird von dem Uhrwerk gesteuert. Der Steuermechanismus weist einen Exzenter 411 auf der Welle 42 des Sekundenrades 11 auf. Mit dem Exzenter 411 greift eine Gabel 43 eines Hemmankers 44 zusammen, der auf einer Ankerwelle 45 schwenkbar gelagert ist und an seinem unteren Arm zwei Anschlagnasen 46 aufweist.

Die Anschlagnasen 46 des Hemmankers 44 wirken mit Flügeln eines Schaltrades 50 zusammen (Fig. 4), das über ein Zahnrad 60 und eine Übersetzung mit dem Ritzel 33 in Verbindung steht, das den Vorschub auf die Zahnstange 34 überträgt.

Durch Hemmung des Schaltrades 50 bei Anschlag eines Flügels gegen eine Anschlagnase 46 des Hemmankers 44 wird jeweils die letzte Getriebestufe des Vorschubwerkes gesperrt und der Vorschub der Zahnstange 34 in kontinuierlich aufeinanderfolgende, gleichmässige Schrittchen aufgeteilt, deren Takt von dem Uhrwerk gesteuert wird.

Zur Erzielung verschiedener umschaltbarer Vorschubgeschwindigkeiten ist der Hemmanker 44 auf seiner Welle 45 axial verstellbar. Zur axialen Verstellung dient eine gewellte Kurvenscheibe 47, die von aussen mittels eines Betätigungsteiles 48 (Fig. 1) von Hand drehbar ist und durch ihren Eingriff in einen Querschlitz 49 in der Ankerwelle 45 eine axiale Verstellung des Hemmankers 44 hervorruft, damit er mit unterschiedlich breiten Flügeln des Schaltrades 50 zusammengreifen kann.

Bei dem gewählten Beispiel sind vier jeweils um 90° zueinander versetzte Flügel an dem Schaltrad 50 vorgesehen. Diese Flügel haben unterschiedliche Breite, und zwar ist der Flügel 51 halb so breit wie der ihm gegenüberliegende Flügel 52, während die beiden anderen Flügel 53 nochmals um die Hälfte schmaler als der Flügel 51 sind. Wird der Hemmanker 44 so eingestellt, dass er bei Umdrehung des Sekundenrades 11 alle vier Flügel 51–53 des Schaltrades 50 hemmt, so wird die Getriebestufe, auf der das Schaltrad 50 sitzt, pro Minute viermal gesperrt, das heisst, es ergeben sich kleine Vorschubstösse, und der vollständige Spritzenweg wird in der Zeit von 24 Stunden erreicht. Bei zwölf Stunden wird nur jeder zweite Flügel (180°) gehemmt, so dass sich ein doppeltgrosser Vorschub ergibt, also zwölf Stunden Laufzeit auf die volle Hublänge. Bei Einstellung einer 6-Stunden-Dauerinfusion wird der Vorschub einmal pro 380° abgestoppt, und es wird eine 6-Stunden-Laufzeit auf 40 mm Vorschublänge erhalten. Eine Umschaltung auf einen anderen Vorschub ist mittels des Beätigungsteiles 48 während des Ablaufes möglich.

In Anpassung an andere Verhältnisse kann die Flügelanordnung des Schaltrades 50 anders als bei dem dargestellten Beispiel gewählt sein.

**Patentansprüche**

1. Gerät für Dauerinfusionen, dessen Laufwerk zum Antrieb einer Injektionsspritze (3) einen Federmechanismus (16, 31, 32) zum Vorschub des Spritzenstössels und ein mechanisches Uhrwerk (8–12) zur Steuerung des Stösselvorschubes (33, 34) aufweist, dadurch gekennzeichnet, dass das Uhrwerk (8–12) den Federmechanismus (16, 31, 32) über Schrittschaltmittel (411, 44, 50) steuert, die die Kraftflüsse des Federmechanismus (16, 31, 32) und des Uhrwerkes (8–12) voneinander trennen.

2. Gerät nach Anspruch 1, dadurch gekennzeichnet, dass der Federmechanismus (16, 31, 32) und das Uhrwerk (8–12) mit je einer Spiralzugfeder (8; 16) und Räderwerkübersetzung (9–11, 31, 32) ausgestattet sind, und dass die beiden Spiralzugfedern (8; 16) koaxial auf einer Federwelle (17) montiert und mit einer gemeinsamen Aufzugswelle (13) verbunden sind.

3. Gerät nach Anspruch 1 oder 2, gekennzeichnet durch ein Gehäuse (1), das eine Auflage (2) für die Injektionsspritze (3) sowie eine schwenkbare Abdeckung (5) für die Spritzenauflage (2) aufweist, wobei die schwenkbare Abdeckung (5) mit der Aufzugswelle (13) drehfest verbunden ist.

4. Gerät nach Anspruch 3, dadurch gekennzeichnet, dass mit der Abdeckung (5) eine schrägverzahnte Scheibe (19) fest verbunden ist, die mit einer Federklinke (29) derart zusammenwirkt, dass die Abdeckung (5) erst nach ihrer Schwenkung um 180° schliessbar ist und dass zur Ausrastung der Federklinke (29) in der End-Schwenkstellung der Abdeckung (5) eine von einem Mitnehmerzapfen (20) an der schrägverzahnten Scheibe (19) betätigte Nockenfläche (30) dient.

5. Gerät nach den Ansprüchen 1 bis 4, dadurch gekennzeichnet, dass der Federmechanismus über eine Übersetzung (32) ein Ritzel (33) antreibt, das eine Zahnstange (34) vorschiebt, die einen an den Stössel (3a) der Spritze (3) angreifenden Arm (4) aufweist, und dass auf der Welle (42) des Sekundenrades (11) des Uhrwerkes ein Exzenter (411) angeordnet ist, der einen auf einer Ankerwelle (45) sitzenden Hemmanker (44) betreibt, welcher auf ein von dem Federmechanismus angetriebenes Schaltrad (50) wirkt, das über die Übersetzung (32) das Ritzel (33) steuert.

6. Gerät nach Anspruch 5, dadurch gekennzeichnet, dass das Schaltrad (50) als Flügelrad mit symmetrisch angeordneten Flügeln (51, 52, 53) ausgebildet ist.

7. Gerät nach Anspruch 6, dadurch gekennzeichnet, dass das Schaltrad (50) vier Flügel (51, 52, 53) mit 90° Abstand aufweist.

8. Gerät nach Anspruch 7, dadurch gekennzeichnet, dass der Hemmanker (44) zwei hin-tereinanderliegende Anschlagnasen (46) für die Flügel (51, 52, 53) des Schaltrades (50) aufweist.

9. Gerät nach den Ansprüchen 6 bis 8, dadurch gekennzeichnet, dass die Breite der Flügel (51–53) zueinander unterschiedlich ist, und dass der Hemmanker (44) zur Erfassung von Flügeln (51–53) unterschiedlicher Breite auf der Ankerwelle (45) axial verstellbar angeordnet ist.

10. Gerät nach Anspruch 9, dadurch gekennzeichnet, dass zur axialen Verstellung des Hemmankers (44) eine gewellte Kurvenscheibe (47) dient, die in einen Querschlitz (49) an der Anker-welle (45) angreift.

11. Gerät nach Anspruch 10, dadurch gekennzeichnet, dass die Stellung der Kurvenscheibe (47) mittels eines Betätigungsteiles (48) auf der Aussenfläche des Gehäuses (1) einstellbar und durch eine Rastvorrichtung gesichert ist.

12. Gerät nach einem der Ansprüche 1 bis 11, dadurch gekennzeichnet, dass die Zahnstange (34) zwischen einem Stangenpaar (41) geführt ist, das an einem Kipphebel (37) befestigt ist, dessen eines Ende schwenkbar mit dem Gehäuse (1) verbunden ist und dessen anderes Ende unter der Wirkung einer Schraubenfeder (39) und einer mit der Aufzugswelle (13) drehfest verbundenen Nokkenscheibe (35) steht.

**Claims**

1. Continuous infusion device having a mechanism for the drive of an injection syringe (3) comprising a spring system (16, 31, 32) to advance the syringe rod, and a mechanical clockwork (8–12) to control the rod advance (33, 34), characterized in that the clockwork (8–12) controls the spring system (16, 31, 32) through step-by-step switching means (411, 44, 50) which separate from each other the flux of force of the spring system (16, 31, 32) and of the clockwork (8–12).

2. Device according to claim 1, characterized in that the spring system (16, 31, 32) and the clockwork (8–12) are provided each with a helical tension spring (8; 16) and a gear transmission (9–11, 31, 32), and that the two helical tension springs (8; 16) are mounted coaxially on a spring shaft (17) and connected to a common winding shaft (13).

3. Device according to claim 1 or 2, characterized by a housing (1) containing a support (2) for the injection syringe (3) and a swivable cover (5) for the syringe support (2), the swivable cover (5) being non-rotatingly connected to the winding shaft (13).

4. Device according to claim 3, characterized in that the cover (5) is inherently connected to a helical toothed disk (19) which cooperates with a spring latch (29) in such a manner that the cover (5) can be closed only upon its swivel by 180° and that for the disengaging of the spring latch (29) in the final swivel position of the cover (5) there is provided a cam face (30) actuated by a tang (20) at the helical toothed disk (19).

5. Device according to claims 1 to 4, characterized in that the spring system drives through a transmission gear (32) a pinion (33) advancing a rack (34), which contains an arm (4) engaging the rod (3a) of the syringe (3), and that on the shaft (42) of the seconds wheel (11) of the clockwork, an eccentric (411) is arranged which operates an anchor escapement (44) seated on an anchor shaft (45), the anchor acting on an indexing wheel (50) driven by the spring system and controlling via the transmission gear (32) the pinion (33).

6. Device according to claim 5, characterized in that the indexing whell (50) is a wing whell having symmetrically arranged wings (51, 52, 53).

7. Device according to claim 6, characterized in that the indexing wheel (4) has four wings (51, 52, 53) at a distance of 90°.

8. Device according to claim 7, characterized in that the anchor escapement (44) contains two consecutive stop noses (46) for the wings (51, 52, 53) of the indexing wheel (50).

9. Device according to claims 6 to 8, characterized in that the width of the wings (51–53) relative to each other is distinctive and that the anchor escapement (44) is mounted to be axially adjustable on the anchor shaft (45) to engage wings (51–53) of varying width.

10. Device according to claim 9, characterized in that for the axial adjustment of the anchor escapement (44), there is provided a corrugated cam plate (47) which engages a transverse slot (49) of the anchor shaft (45).

11. Device according to claim 10, characterized in that the position of the cam plate (47) is adjustable by means of an actuating member (48) on the outside of the housing (1) and is secured by a stop means.

12. Device according to one of claims 1 to 11, characterized in that the rack (34) is guided between a pair of bars (41) secured to a rocker arm (37) whose one end is hinge-connected to the housing (1) and whose other end is under the action of a coil spring (39) and of a cam plate (35) non-rotatingly connected to the winding shaft (13).

## Revendications

1. Appareil pour injections permanentes, dont le dispositif d'entraînement d'une seringue d'injection (3) comporte un mécanisme à ressort (16, 31, 32) pour faire avancer le poussoir de seringue et une minuterie mécanique (8–12) pour la commande de l'avance du poussoir (33, 34) caractérisé en ce que la minuterie (8–12) commande le mécanisme à ressort (16, 31, 32) par l'intermédiaire de moyens d'entraînement pas à pas (411, 44, 50) qui coupent les transmissions de force entre le mécanisme à ressort (16, 31, 32) et la minuterie (8–12).

2. Appareil selon la revendication 1, caractérisé en ce que le mécanisme à ressort (16, 31, 32) et la minuterie (8–12) sont chacun équipés d'un ressort spiral de traction (8; 16) et d'une transmission à engrenages (9–11, 31, 32) et en ce que les deux ressorts spiraux de traction (8; 16) sont montés coaxialement sur un axe de ressort (17) et sont reliés à un arbre de mise en tension commun (13).

3. Appareil selon l'une des revendications 1 ou 2, caractérisé en ce qu'il est prévu un carter (1) qui comporte un support (2) pour la seringue d'injection (3) ainsi qu'un couvercle pivotant (5) pour le support de seringue (2), le couvercle pivotant (5) étant relié sans possibilité de rotation relative avec l'arbre de mise en tension (13).

4. Appareil selon la revendication 3, caractérisé en ce que le couvercle (5) est relié rigidement à un disque (19) pourvu d'une denture inclinée et qui coopère avec un cliquet élastique (29) de manière que le couvercle (5) puisse être fermé seulement après son pivotement de 180° et en ce que, pour désaccoupler le cliquet élastique (29) dans la position limite de pivotement du couvercle (5) il est prévu une surface de came (30) actionnée par un téton d'entraînement (20) prévu sur le disque à denture inclinée (19).

5. Appareil selon l'une des revendications 1 à 4, caractérisé en ce que le mécanisme à ressort entraîne par l'intermédiaire d'une transmission (32) un pignon (33) qui fait avancer une crémaillère (34) qui comporte un bras (4) s'accrochant sur le poussoir (3a) de la seringue (3) et en ce qu'il est prévu sur l'axe (42) de la roue des secondes (11) de la minuterie un excentrique (411) qui actionne une ancre d'échappement (44) montée sur une tige d'ancre (45) et agissant sur une roue de commande (50) entraînée par le mécanisme à ressort et commandant le pignon (33) par l'intermédiaire de la transmission (32).

6. Appareil selon la revendication 5, caractérisé en ce que la roue de commande (50) est agencée sous la forme d'une roue à palettes comportant des palettes (51, 52, 53) disposées symétriquement.

7. Appareil selon la revendication 6, caractérisé en ce que la roue de commande (50) comporte quatre palettes (51, 52, 53) espacées de 90°.

8. Appareil selon la revendication 7, caractérisé en ce que l'ancre d'échappement (44) comporte deux ergots de butée (46) placés l'un derrière l'autre pour les palettes (51, 52, 53) de la roue de commande (50).

9. Appareil selon l'une des revendications 6 à 8, caractérisé en ce que les largeurs des palettes (51–53) sont différentes entre elles et en ce que l'ancre d'échappement (44) est montée sur la tige d'ancre (45) de façon à pouvoir se déplacer axialement pour retenir les palettes (51, 53) de largeurs différentes.

10. Appareil selon la revendication 9, caractérisé en ce que, pour le déplacement axial de l'ancre d'échappement (44) il est prévu une came ondulée (47) qui s'accroche dans une fente transversale (49) de la tige d'ancre (45).

11. Appareil selon la revendication 10, caractérisé en ce que la position de la came (47) est réglable à l'aide d'une partie d'actionnement (48) sur la surface extérieure du carter (1) et peut être bloquée à l'aide d'un dispositif d'arrêt.

12. Appareil selon l'une des revendications 1 à 11, caractérisé en ce que la crémaillère (34) est guidée entre deux barres (41) qui sont fixées sur un levier basculant (37), dont une extrémité est reliée à pivotement au carter (1) et dont l'autre extrémité est soumise à l'action d'un ressort hélicoïdal (39) et d'une came (35) reliée sans possibilité de rotation relative à l'axe de tension (13).

FIG.1

0 016 343

FIG.2

FIG.3

FIG.3a

FIG.4

0 016 343

FIG.5

FIG.6

FIG.7

FIG.8

13